# EUROPÄISCHE PATENTANMELDUNG

(11) **EP 1 099 449 A1**
(43) Veröffentlichungstag der Anmeldung: **16.05.2001**
(21) Anmeldenummer: 00810841.7
(22) Anmeldetag: 18.09.2000
(51) Int. Cl.: A61M 5/315, B29C 45/16

(54) **Verfahren zur Herstellung eines Pumpkolbens für eine Einwegspritze und ein Pumpkolben**

(30) Priorität: 12.11.1999 CH 206899
(71) Anmelder: SCHÖTTLI AG, 8253 Diessenhofen (CH)
(72) Erfinder: Schottli, Martin, 8254 Basadingen (CH)
(74) Vertreter: Gachnang, Hans Rudolf

(57) **Zusammenfassung**

Der Pumpkolben (9) einer Einwegspritze wird im Zweikomponenten-Verfahren hergestellt. Das Einspritzen des Kunststoffs für das Dichtelement (33) erfolgt radial durch eine Bohrung, welche beim Spritzen des Schafts (21) durch eine Nadel (41) ausgespart worden ist. Der erfindungsgemässe Pumpkolben (9) ist nach dem Spritzen einbaufertig; es müssen keine weiteren Montagearbeiten daran vorgenommen werden.

## Beschreibung

Gegenstand der Erfindung ist ein Verfahren zur Herstellung eines Pumpkolbens für eine Einwegspritze gemäss Oberbegriff des Patentanspruchs 1. Gegenstand der Erfindung ist weiter ein nach dem Verfahren hergestellter Pumpkolben für eine Einwegspritze gemäss Oberbegriff des Patentanspruchs 4.

Einwegspritzen werden in Spitälern und Arztpraxen in grosser Zahl benötigt. Sie sind in unzähligen Ausführungsformen als zwei- oder dreiteilige Kunststoffspritzen bekannt. Die zweiteiligen Spritzen umfassen einen Pumpkolben mit einer Kolbenstange und einer Pressplatte. Der Spritzenzylinder weist an seinem einen Ende einen Nadelkonus und an seinem anderen Griffplatten auf. Die Abdichtung zwischen Kolben und Zylinder erfolgt durch einen gegenüber dem Innendurchmesser des Zylinders etwas grösseren Durchmesser des Kolbens.

Es sind auch Pumpkolben bekannt, an deren Peripherie elastische Lippen ausgebildet sind.

Bei den dreiteiligen Spritzen sitzt auf dem Kolben zusätzlich ein Dichtungsring aus einem Elastomer, der nachträglich, d.h. nach der Fertigstellung des Pumpkolbens und vor dessen Einführen in den Zylinder aufgesetzt werden muss. Dreiteilige Einwegspritzen haben den Vorteil, dass die Reibung zwischen dem Kolben und dem Zylinder kleiner ist, so dass ein gleichmässiges Vorschieben des Kolbens beim Einspritzens sichergestellt werden kann.

Es ist auch bekannt, die Zylinderinnenfläche, d.h. die Lauffläche für den Kolben, zu silikonisieren, um ein ruckfreies gleichmässiges Gleiten des Kolbens sicherzustellen. Auf diese Weise gelangt aber das verwendete Silikongleitmittel in kleinen Mengen in den Körper des Menschen oder des Tieres. Dies ist auf jeden Fall unerwünscht.

Die Aufgabe der vorliegenden Erfindung ist die Schaffung eines kostengünstig herstellbaren Pumpkolbens für eine Einwegspritze sowie eines Verfahrens zur Herstellung eines Pumpkolbens für eine Spritze.

Gelöst werden diese Aufgaben durch ein Verfahren gemäss den Merkmalen des Patentanspruchs 1 sowie einem Pumpkolben gemäss Patentanspruch 4.

Der Pumpkolben kann in zwei Schritten, jedoch innerhalb derselben Spritzform aus zwei unterschiedlichen, funktionsmässig geeigneten Materialien gespritzt werden.
Die Herstellung eines solchen Pumpkolbens ist sehr kostengünstig, und es müssen keine manuellen Montage-Tätigkeiten, nämlich Aufsetzen eines Dichtungsrings, ausgeführt werden. Der die Dichtung bildende Weichkunststoffteil kann nicht vom Kolben abspringen, so dass dessen Montage in den Zylinder ohne nachträgliche Überprüfung/Kontrolle möglich ist. Die Dichtung ist auf jeden Fall gewährleistet.

Anhand illustrierter Ausführungsbeispiele wird die Erfindung näher erläutert. Es zeigen
- Figur 1: eine perspektivische Darstellung einer Einwegspritze mit teilweise eingeschobenem Pumpkolben,
- Figur 2: einen Querschnitt durch ein Werkzeug zum Spritzen von Pumpkolben für Einwegspritzen (direkte Einspritzung der zweiten Komponente),
- Figuren 3-6: je eine Herstellungsstufe beim Spritzen der zweiten Komponente durch die erste Komponente hindurch,
- Figur 7: einen Schnitt durch den Pumpkolben in einer weiteren Ausgestaltung eines Pumpkolbens,
- Figur 8: einen Querschnitt durch den vorderen Teil eines Pumpkolbens, hergestellt in einem Werkzeug gemäss Figur 7,
- Figur 9: einen Schnitt durch den vorderen Teil eines Pumpkolbens in einer weiteren Ausgestaltung der Erfindung und
- Figur 10: eine Aufsicht auf den Pumpkolben in Figur 9.

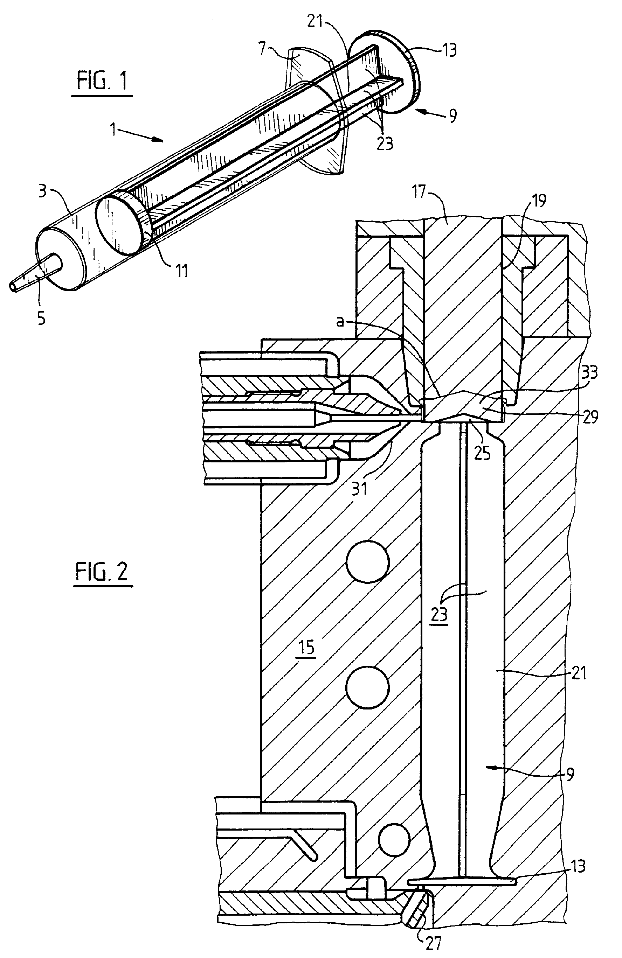

Die Einwegspritze 1 in Figur 1 umfasst einen Pumpzylinder 3 an dessen unteren Ende ein Nadelkonus 5 ausgebildet ist und an dessen oberen Ende radial abstehende Griffplatten 7 angeformt sind. In den Zylinder 3 ist ein Pumpkolben 9 eingeschoben, auf dessen vorderen Ende eine Dichtung 11 angebracht ist und dessen hinteres Ende eine Druckplatte 13 aufweist.

Die nachfolgend beschriebene Erfindung betrifft nur den Pumpkolben 9; der Zylinder 3 und dessen Ausbildung werden als bekannt vorausgesetzt und nicht näher erläutert.

Der erfindungsgemässe Pumpkolben 9 umfasst einen Schaft 21, der aus vier sternförmig angeordneten Rippen 23 bestehen kann, einem Konus oder Kegel 25 am vorderen Ende und der Griff- oder Druckplatte 13 am hinteren Ende.

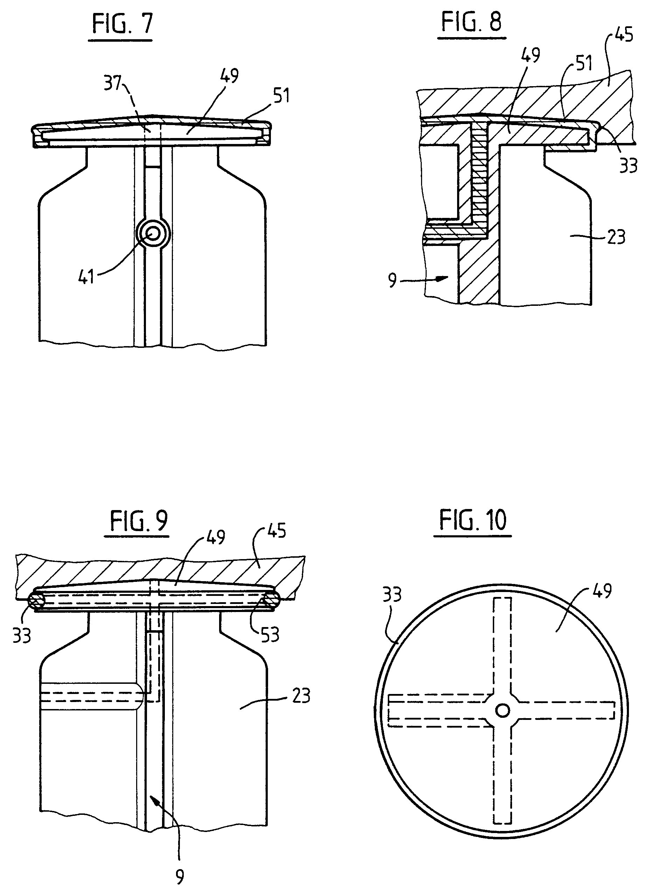

Mit dem Konus 25 wird eine Dichtung unlösbar verbunden. Letztere kann den Konus 25 ganz oder teilweise (Fig. 7/8) umgeben oder die Gestalt eines O-Ringes (Figur 9) aufweisen.

Nachfolgend wird anhand der Figuren 2 bis 10 die Herstellung des Pumpkolbens 9 erläutert.

In den Figuren 2 bis 5 werden im Werkzeug die verschiedenen Bereiche der Hohlräume mit den gleichen Bezeichnungen wie die Teile des Pumpkolbens benannt.

In einem Spritzwerkzeug 15, in welchem der Pumpkolben 9 hergestellt wird, ist ein Schieber 17 in Gestalt eines Zylinders eingebaut und in einer Bohrung 19 im Spritzwerkzeug 15 axial zur Längsachse des Pumpkolbens 9 verschiebbar ist. Die Antriebsorgane für den Schieber 17 sind nicht dargestellt. Bei vorgeschobenem Schieber 17, wie er in Figur 2 dargestellt ist und wie er zu Beginn des Spritzvorgangs vorliegt, ist die Kavität im Werkzeug 15 derart gestaltet, dass der Pumpkolben 9 entsteht, an dessen vorderen Ende die vier Rippen 23 des Schafts 21 den flachen Konus 25 als Dichtelementaufnahme tragen. Die Einspritzung des flüssigen Kunststoffs in die Kavität für den Pumpkolben 9 erfolgt mit einer Düse 27, welche Kunststoff im Bereich der Druckplatte 13 einspritzt, die das untere Ende des Schafts 21 bildet.

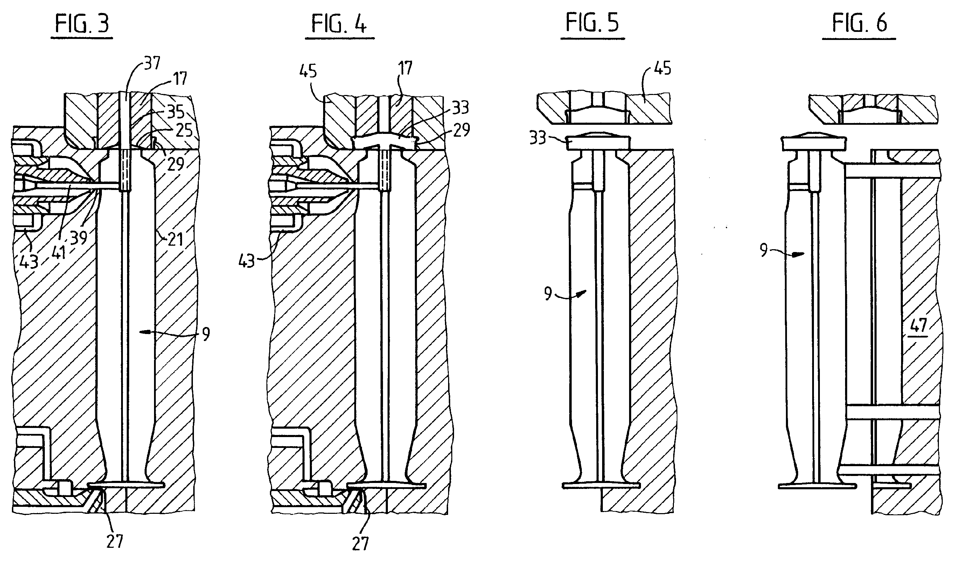

Nach dem Einspritzen des Kunststoffs für den Schaft 21, die Druckplatte 13 und den Konus 25 mit der ersten Düse 27 und nach einer kurzen Erstarrungspause wird der Schieber 17 axial zurückgezogen und dadurch über dem Konus 25, der den Schaft 21 abschliesst, eine neue Kavität 29 geschaffen (Umriss in feinen Linien in Figur 2). In diese zusätzliche Kavität 29 wird durch eine zweite Düse 31 eine zweite Kunststoffkomponente mit vorzugsweise hoher Elastizität und guten Gleiteigenschaften eingespritzt. In der zusätzlichen Kavität 29 entsteht dann ein Dichtelement 33, z.B. eine Dichtungsplatte bzw. ein Dichtungsring, welches unlösbar mit dem Konus 25 des Pumpkolbens 9 verbunden ist. Nach der Erstarrung des das Dichtelement 33 bildenden Kunststoffs kann der Pumpkolben 9 ausgeformt werden. Der so erzeugte Pumpkolben 9 ist ohne weitere Bearbeitung in den Zylinder 3 der Einwegspritze 1 einsetzbar. Das in den Figuren 4 bis 6 eine zylindermantelförmige Peripherie aufweisende Dichtelement 33 kann selbstverständlich auch mit umlaufenden Rillen (Labyrinthen) versehen werden.

In der zweiten Ausgestaltung der Erfindung für die Erzeugung des Dichtelementes 33, z.B. der Dichtungsplatte bzw. des Dichtungsrings am Ende des Pumpkolbens 9, ist im Schieber 17 eine axiale Nadelbohrung 35 ausgebildet, durch welche eine Nadel 37 axial in die Kavität 21 für den Pumpkolben 9 einschiebbar ist. In einem rechten Winkel zur ersten Nadelbohrung 35 ist eine zweite Nadelbohrung 39 in einem Abstand zum Ende der Kavität 21 ausgebildet, in welche eine zweite Nadel 41, die Teil einer Einspritzdüse 43 ist, geführt wird und in die Kavität 21 einschiebbar ist.

Während des Spritzens des Schafts 21 des Pumpkolbens 9 sind beide Nadeln 37 und 41 vorgeschoben und berühren einander. Dadurch entsteht im Schaft 21 des Pumpkolbens 9 eine rechtwinklig verlaufende Ausnehmung. Diese dient im zweiten Verfahrensschritt der Herstellung gemäss Figur 4 dazu, in die zusätzliche Kavität 29 nach dem Zurückziehen des Schiebers 17 mit der Düse 43 die zweite Kunststoffkomponente für die Erzeugung des Dichtelements einzuspritzen. Nach der Fertigstellung des Pumpkolbens 9 sind die anfänglich durch die vorgeschobenen Nadeln 37 und 41 im Schaft 21 gebildeten Kanäle mit dem Material, aus dem das Dichtelement 33,51 besteht, gefüllt.

Zum Ausformen des aus zwei Komponenten bestehenden Pumpkolbens 9 wird zum einen der den Schieber 17 enthaltende Werkzeugteil 45 nach oben weggeführt und danach der in den Figuren 3 bis 6 rechts der Kavität 27 liegende Werkzeugteil 47 zur Seite bewegt.

Auch dieser Pumpkolben 9 ist nach der Ausformung ohne weitere Bearbeitung in den Zylinder 3 der Einwegspritze 1 einschiebbar.

Die Dichtplatten bzw. Dichtringe 33 in den ersten beiden Ausführungsbeispielen gemäss den Figuren 2 bis 6 bestehen vollständig aus der zweiten elastischen Komponente, welche auf den Konus 25 am Ende des Schafts aufgespritzt ist.

In den Figuren 7 bis 10 wird ein Pumpkolben 9 dargestellt, bei dem die elastische Komponente für das Dichtelement als Überzug (Figuren 7 und 8) oder als umlaufender Ring (Figuren 9 und 10) ausgebildet ist.

In beiden Ausführungsformen wird vorerst am vorderen Ende der Rippen 23 im ersten Arbeitsgang eine scheibenförmige Platte 49 ausgebildet. Anschliessend wird der Schieber 17 um wenige Hundertstelsmillimeter zurückgezogen und die zweite Kunststoffkomponente durch die Bohrungen, erzeugt durch die beiden Nadeln 37 und 41 (wie in den Figuren 3 und 4 ersichtlich), zugeführt. Dadurch entsteht ein dünnes Dichtelement, d.h. eine sehr elastische Dichtungshaut 51, welche die Platte 49 vollständig überzieht. Durch geeignete Formgebung des oberen Werkzeugteils 45 bildet sich an der Peripherie der Platte 49 ein wulstförmiger Dichtungsring 33.

In der Ausgestaltung der Erfindung gemäss den Figuren 9 und 10 ist in der Peripherie der Platte 49 im ersten Verfahrensschritt eine umlaufende Rille 53 erzeugt worden, welche im zweiten Verfahrensschritt durch die zweite weiche Kunststoffkomponente gefüllt und ein Dichtungsring 33 gebildet wird, wie er als O-Ring bekannt ist. Der Dichtungsring 33 ist jedoch unlösbar mit dem Pumpkolben 9 verbunden und muss nicht nachträglich montiert werden.

## Patentansprüche

1. Verfahren zur Herstellung eines Pumpkolbens (9) für eine Einwegspritze (1) aus Kunststoff, bei dem der Schaft (21) und die Druckplatte (13) an einem Ende sowie eine Dichtringaufnahme (25,49) am andern Ende des Schafts (21) aus einer ersten Kunststoffkomponente mit einer ersten Einspritzdüse (27) gespritzt und innerhalb des gleichen Werkzeugs (15) nach Vergrösserung der Kavität (29) anschliessend auf das vordere Ende ein elastisches Dichtelement (33,51) aus einer zweiten Kunststoffkomponente aufgespritzt wird.

2. Verfahren nach Anspruch 1, dadurch gekennzeichnet, dass das Einspritzen des Kunststoffs für das Dichtelement (33,51) mit einer zweiten Einspritzdüse (31,43) durch eine im Schaft (21) des Pumpkolbens (9) bei dessen Herstellung ausgesparte Ausnehmung (39) erfolgt.

3. Verfahren nach Anspruch 1, dadurch gekennzeichnet, dass das Einspritzen des Kunststoffs für das Dichtelement (33,51) mit einer zweiten Einspritzdüse (43) direkt in die vergrösserte Kavität (29) erfolgt.

4. Pumpkolben (9) aus Kunststoff für eine Einwegspritze (1), hergestellt nach dem Verfahren nach Anspruch 1, mit einem Spritzenzylinder (3), an deren einem Ende ein Nadelkonus (5) und an dessen anderen Ende mindestens eine Griffplatte (7) ausgebildet sind, und einem Dichtelement (33,51) am vorderen Ende des Schafts (21) des Pumpkolbens (9), dadurch gekennzeichnet, dass das Dichtelement (33,51) aus einem Material besteht, das eine höhere Elastizität aufweist als das Pumpkolbenmaterial und im gleichen Werkzeug nach dem Spritzen des Pumpkolbens (9) an diesen unlösbar angespritzt ist.

5. Pumpkolben (9) nach Anspruch 4, dadurch gekennzeichnet, dass am vorderen Ende des Schafts (21) ein Konus (25) oder eine scheibenförmige Platte (49) ausgebildet ist, welche das Dichtelement (33,51) trägt.

6. Pumpkolben (9) nach Anspruch 5, dadurch gekennzeichnet, dass das Dichtelement (33,51) die scheibenförmige Platte (49) vollständig umgibt bzw. letztere überzieht.

7. Pumpkolben (9) nach Anspruch 5, dadurch gekennzeichnet, dass das Dichtelement (33,51) mit der Peripherie der Platte (49) verbunden ist.

8. Pumpkolben (9) nach Anspruch 5, dadurch gekennzeichnet, dass das Dichtelement (33) in einer umlaufenden Nut (53) in der Peripherie der Platte (49) ausgebildet ist und einen runden Querschnitt aufweist.

9. Pumpkolben (9) nach einem der Ansprüche 4 bis 8, dadurch gekennzeichnet, dass der Schaft (21) mindestens einen radial durch eine Rippe (23) verlaufenden ersten Einspritzkanal, gefüllt mit dem Kunststoffmaterial für das Dichtelement (33,51), aufweist.

10. Pumpkolben nach einem der Ansprüche 4 bis 9, dadurch gekennzeichnet, dass der Schaft (21) einen axial verlaufenden zweiten Einspritzkanal, gefüllt mit Kunststoffmaterial für das Dichtelement (33,51), aufweist.
